# EUROPEAN PATENT APPLICATION

(11) **EP 2 821 011 A1**
(43) Date of publication of application: **07.01.2015**
(21) Application number: 13755209.7
(22) Date of filing: 26.02.2013
(51) Int. Cl.: A61B 6/00

(54) **PERIODIC PATTERN DETECTION DEVICE AND METHOD**

(30) Priority: 27.02.2012 JP 2012039591; 28.02.2012 JP 2012040891
(71) Applicant: FUJIFILM Corporation, Tokyo 106-8620 (JP)
(72) Inventor: IMAI, Yoshiro, Ashigarakami-gun Kanagawa 258-8538 (JP)
(74) Representative: Klunker . Schmitt-Nilson . Hirsch
(86) International application number: PCT/JP2013/001101
(87) International publication number: WO 2013/128890

(57) **Abstract**

[Problems to be Solved]

When an area in which it is difficult to detect a periodic pattern is further included in a radiation field area, allowing a periodic pattern to be detected accurately.

[Means for Solving the Problems]

The image obtaining unit (31) obtains a radiation image (P0) from the radiation detector (2), the radiation field area detection unit (32) detects a radiation field area (P1) from the radiation image (P0). The analysis area setting unit (33) detects a direct radiation area, a superabsorbent body area, and a high noise area as the areas where a periodic pattern is unlikely to present and sets an analysis area (P2) by excluding these areas. The frequency analysis unit (34) performs a frequency analysis only on the analysis area (P2) and detects a frequency component of the periodic pattern arising from a grid. The filtering processing unit (35) removes the frequency component of the periodic pattern arising from the grid from the radiation image (P0).

## Description

### BACKGROUND OF THE INVENTION

### Technical Field

The present invention relates to a periodic pattern detection apparatus and method that, in suppressing a periodic pattern included in an image, detects a frequency component of the periodic pattern.

### Background Art

Various types of radiation detectors that record radiation images of subjects by receiving radiation transmitted through the subjects have been proposed and put into practical use in the field of medicine and the like. Such radiation detectors include, for example, a radiation detector that uses amorphous selenium that generates an electric charge by receiving radiation. In an imaging system that uses the radiation detector, an anti-scatter grid (hereinafter, simply "grid") having lead or the like that does not transmit radiation and aluminum or wood that easily transmits radiation disposed alternately at a predetermined pitch is provided between a radiation source that emits radiation and the radiation detector, and a scatted component of the radiation is removed by the grid.

If a radiation image of a subject is captured using a grid, however, a frequency component of a periodic pattern, such as a periodic stripe pattern, a moire pattern, and the like arising from the grid appears in the obtained image as noise. Consequently, a variety of proposals for preventing image quality degradation arising from the periodic pattern have been proposed in which periodic patterns included in radiation images are detected and processing for suppressing frequency components of the periodic patterns is performed on the radiation images. For example, Patent Document 1 proposes a method in which a plurality of linear areas is set in a radiation image, then a frequency spectrum is obtained by performing a frequency analysis, such as Fourier transformation or the like, on the image signals of the linear areas, and a frequency having a peak response in the frequency spectrum is detected as the frequency component of the periodic pattern.

Further, when detecting the frequency component of the period pattern in the manner described above, the method described in Patent Document 1 sets a plurality of subareas, calculates a frequency spectrum in each subarea, and calculates an average value of frequency spectra of all of the subareas. By calculating the average value of frequency spectra in the manner described above, the frequency component of the period pattern may be detected accurately without being influenced by noise.

In the meantime, if the density of the grid is relatively high, the amplitude of the periodic pattern is weak. Further, as the frequency component of the period pattern is folded back more to low-frequency, the frequency component is more likely to be buried in the frequency components of the subject. In such a case, the response of the frequency component of the periodic pattern in the frequency spectrum is small and the frequency component of the periodic pattern is difficult to detect.

In the meantime, when performing imaging, a radiation field stop is sometimes used in order to prevent radiation from being projected on an unnecessary region of the subject. If the subject is imaged using the radiation field stop, no periodic pattern is included in the area outside the radiation field in the radiation image since the radiation was not projected thereon. Further, if imaging is performed using a protector, such as radiation absorbing lead or the like, in order to prevent radiation from being projected on an unnecessary region of the subject, such as genitals and the like, the protector area (hereinafter, "superabsorbent body area") in the radiation image has low density and the periodic pattern is barely noticeable.

Consequently, a method that detects a periodic pattern only from the portion of a radiation image corresponding to a radiation field area (Patent Document 2) and a method that removes a periodic pattern by setting the image signal value in the portion of a radiation image where a protector is present to a value of 0 (Patent Document 3) are proposed. Further, a method that removes noise from a radiation image other than the area in the radiation image where the radiation was projected directly is proposed (Patent Document 4) .

### [Prior Art Documents]

### [Patent Documents]

Patent Document 1: Japanese Unexamined Patent Publication No. 2003-233818
Patent Document 2: Japanese Unexamined Patent Publication No. 2002-325765
Patent Document 3: Japanese Unexamined Patent Publication No. 2003-037777
Patent Document 4: Japanese Unexamined Patent Publication No. 2010-009348

### DISCLOSURE OF THE INVENTION

The methods described in Patent Documents 2 to 4, however, simply detect and remove periodic patterns by excluding the outside radiation field area, the superabsorbent body area, and the direct radiation area, and are unable to deal with if an area where it is difficult to detect a periodic pattern is further included in the radiation field area.

Further, if the density of the grid is relatively high, the amplitude of the periodic pattern is weak. Still further, as the frequency component of the period pattern is folded back more to low-frequency, the frequency component is more likely to be buried in the frequency components of the subject. In such a case, the response of the frequency component of the periodic pattern in the frequency spectrum is small and the frequency component of the periodic pattern is difficult to detect.

In the meantime, when performing imaging, a radiation field stop is sometimes used in order to prevent radiation from being projected on an unnecessary region of the subject. If the subject is imaged using the radiation field stop, no periodic pattern is included on the area outside the radiation field in the radiation image since the radiation was not projected thereon. Further, if imaging is performed using a protector, such as radiation absorbing lead or the like, in order to prevent radiation from being projected on an unnecessary region of the subject, such as genitals and the like, the protector area (hereinafter, "superabsorbent body area") in the radiation image has low density and the periodic pattern is barely noticeable. For such an area where periodic pattern is barely noticeable, the calculation of frequency spectrum does not allow accurate detection of frequency component of the periodic pattern.

The present invention has been developed in view of the circumstances described above, and it is an object of the present invention allow, when an area in which it is difficult to detect a period pattern is further included in a radiation field area, accurate detection of the periodic pattern.

It is another object of the present invention to allow, when an area where a periodic pattern is unnoticeable is included in a radiation image, accurate detection of a frequency component of the periodic pattern.

A first periodic pattern detection apparatus of the present invention includes:
a radiation field area detection means that detects, from a radiation image obtained by performing imaging using a radiation field stop and a grid that removes a scattered component of radiation, a radiation field area corresponding to the radiation field stop;
an analysis area setting means that sets an analysis area for performing a frequency analysis by excluding an area in the radiation field area where a periodic pattern is unlikely to present; and
a frequency analysis means that detects a frequency component of the periodic pattern by performing a frequency analysis on the analysis area.

Here, the radiation image may be any image as long as it is detected by projecting radiation on a subject. For example, it may be a radiation image obtained by mammography equipment, a radiation image obtained by chest imaging, or a radiation image used for non-destructive test other than medical images.

Further, the radiation image may be obtained by the use of a radiation detector or a storage phosphor sheet that makes use of a storage phosphor body that stores, by receiving radiation, a part of the energy of the radiation and, thereafter, emits stimulated emission light by receiving excitation light, such as visible light, laser light, or the like. In the case where the storage phosphor sheet is used, a radiation image is obtained by tentatively storing and recording radiation image information by projecting radiation transmitted through a subject on the storage phosphor sheet, generating stimulated emission light by projecting excitation light on the storage phosphor sheet, and photoelectrically converting the stimulated emission light.

The grid may have any pattern as long as it removes a scattered component of radiation and, for example, it may be formed of a plurality of plates provided along the main or sub direction of the radiation detector or formed of a plurality of plates tilted with respect to the main and sub directions of the radiation detector.

The periodic pattern refers to noise having a periodic pattern in a radiation image. For example, it refers to a periodic stripe pattern, a moire pattern, or the like included in a radiation image obtained by imaging a subject using a grid.

In the first periodic pattern detection apparatus of the present invention, the area where the periodic pattern is unlikely to present may be at least one of a direct radiation area, a superabsorbent body area, and a high noise area.

The direct radiation area refers to a high density area in a radiation image obtained by the radiation projected onto a subject and reached directly on a radiation detector or the like without transmitting through the subject.

The superabsorbent body area refers to a low density area in a radiation image when the imaging is performed using a protector for preventing the radiation from being projected on an unnecessary region of the subject, such as genitals and the like.

Here, if imaging is performed with a low dose of radiation, quantum noise of the radiation is noticeable in a relatively low density area. The high noise area refers to an area where such quantum noise of radiation is noticeable.

In the first periodic pattern detection apparatus of the present invention, the analysis area setting means may set the analysis area according to an imaging condition at the time of obtaining the radiation image.

The imaging condition may be information from which the dose of radiation projected on a subject can be estimated, such as a tube voltage for the radiation source that emits radiation, a mAs value (i.e., electric currentxexposure time), SID (distance from the radiation source to the detection surface of the radiation), an imaging region, and the like.

Further, in the first periodic pattern detection apparatus of the present invention, the area where the periodic pattern is unlikely to present may be a high noise area.

Still further, the first periodic pattern detection apparatus of the present invention may further includes a processing means that obtains a processed radiation image by removing the frequency component of the periodic pattern.

A first periodic pattern detection method of the present invention includes the steps of:
detecting, from a radiation image obtained by performing imaging using a radiation field stop and a grid that removes a scattered component of radiation, a radiation field area corresponding to the radiation field stop;
setting an analysis area for performing a frequency analysis by excluding an area in the radiation field area where a periodic pattern is unlikely to present; and
detecting a frequency component of the periodic pattern by performing a frequency analysis on the analysis area.

A second periodic pattern detection apparatus of the present invention includes:
an area setting means that sets a plurality of subareas throughout a radiation image obtained by performing imaging using a grid that removes a scattered component of radiation;
a presence degree setting means that sets a presence degree of a periodic pattern arising from the grid to each of the plurality of subareas; and
a frequency analysis means that calculates a plurality of frequency spectra for each of the plurality of subareas by performing a frequency analysis on each of the plurality of subareas and detects a frequency component of the periodic pattern by weighted averaging the plurality of frequency spectra according to the presence degree.

In the second periodic pattern detection apparatus of the present invention, if a subarea belongs to at least one of an outside radiation field area, a direct radiation area, a superabsorbent body area, and a high noise area in the radiation image, the presence degree setting means may be a means that sets the presence degree of the subarea smaller than that of a subarea which belongs to an area other than the outside radiation field area, the direct radiation area, the superabsorbent body area, and the high noise area.

The outside radiation field area refers to an area in a radiation image obtained as a result that no radiation was projected on a radiation detector or a storage phosphor sheet (hereinafter, referred to as the radiation detector) when a subj ect was imaged using a radiation field stop.

The term "belongs" refers to not only the entire subarea is included in at least one of an outside radiation field area, a direct radiation area, a superabsorbent body area, and a high noise area in the radiation image but also a part of the subarea is included in at least of these areas.

In the second periodic pattern detection apparatus of the present invention, if a subarea belongs to a high noise area, the presence degree setting means may be a means that sets the presence degree of the subarea smaller than that of a subarea which belongs to an area other than the high noise area.

In the second periodic pattern detection apparatus of the present invention, the presence degree setting means may be a means that sets the presence degree according to an imaging condition at the time of obtaining the radiation image.

The second periodic pattern detection apparatus of the present invention may further includes a processing means that obtains a processed radiation image by removing the frequency component of the periodic pattern.

A second periodic pattern detection method of the present invention includes the steps of:
setting a plurality of subareas throughout a radiation image obtained by performing imaging using a grid that removes a scattered component of radiation;
setting a presence degree of a periodic pattern arising from the grid to each of the plurality of subareas;
calculating a plurality of frequency spectra for each of the plurality of subareas by performing a frequency analysis on each of the plurality of subareas; and
detecting a frequency component of the periodic pattern by weighted averaging the plurality of frequency spectra according to the presence degree.

According to the first periodic pattern detection apparatus and method of the present invention, an analysis area for performing a frequency analysis is set by excluding an area in a radiation field area where a periodic pattern due to a grid is unlikely to present, and a frequency component of the periodic pattern is detected by performing a frequency analysis on the analysis area. Consequently, even when a portion in which the amplitude of a period pattern is weak and a frequency response is small in a frequency spectrum obtained by the frequency analysis is included in the radiation field area, that portion is not used in the frequency analysis. Therefore, the frequency component of the periodic pattern may be detected accurately without being influenced by the area in which a periodic pattern is unlikely to present.

Further, by setting the analysis area according to the imaging condition, the analysis area may be set appropriately according to the circumstances in which the radiation image was obtained. Therefore, the frequency component of the periodic pattern may be detected more accurately.

According to the second periodic pattern detection apparatus and method of the present invention, a plurality of subareas is set throughout a radiation image obtained by performing imaging using a grid that removes a scattered component of radiation, a presence degree of a periodic pattern arising from the grid is set to each of the plurality of subareas, and a plurality of frequency spectra is calculated for each of the plurality of subareas by performing a frequency analysis on each of the plurality of subareas. Then, a frequency component of the periodic pattern is detected by weighted averaging the plurality of frequency spectra according to the presence degree. Consequently, even when a portion in which the amplitude of a period pattern is weak and a frequency response is small in a frequency spectrum obtained by the frequency analysis is included in the radiation field area, the weight of that portion is reduced when detecting the frequency component. Therefore, the frequency component of the periodic pattern may be detected accurately without being influenced by the area where a periodic pattern is unlikely to present.

Further, by setting the presence degree according to the imaging condition, the analysis area may be set appropriately according to the circumstances in which the radiation image was obtained. Therefore, the frequency component of the periodic pattern may be detected more accurately.

### BRIEF DESCRIPTION OF DRAWINGS

Figure 1 is a schematic block diagram of a radiation image diagnostic system to which a periodic pattern detection apparatus according to a first embodiment of the present invention is applied, illustrating a configuration thereof.
Figure 2 is a schematic block diagram of an image processing apparatus, illustrating a configuration thereof.
Figure 3 illustrates an example radiation image.
Figure 4 illustrates an example radiation field area.
Figure 5 illustrates a histogram of signal values of the radiation field area.
Figure 6 illustrates subareas for frequency analysis.
Figure 7 illustrates an example frequency spectrum.
Figure 8 illustrates subareas used for frequency analysis.
Figure 9 illustrates a frequency component of a periodic pattern.
Figure 10 illustrates a frequency spectrum of a processed radiation image.
Figure 11 is a flowchart illustrating processing performed in the first embodiment.
Figure 12 is a schematic block diagram of an image processing apparatus in a radiation image diagnostic system to which a periodic pattern detection apparatus according to a second embodiment of the present invention is applied, illustrating a configuration thereof.
Figure 13 is a flowchart illustrating processing performed in the second embodiment.
Figure 14 is a schematic block diagram of an image processing apparatus in a radiation image diagnostic system to which a periodic pattern detection apparatus according to a third embodiment of the present invention is applied, illustrating a configuration thereof.
Figure 15 illustrates an example radiation image in the third embodiment.
Figure 16 is a drawing for explaining the presence degree according to the area where subareas belong.
Figure 17 is a flowchart illustrating processing performed in the third embodiment.
Figure 18 is a schematic block diagram of an image processing apparatus in a radiation image diagnostic system to which a periodic pattern detection apparatus according to a fourth embodiment of the present invention is applied, illustrating a configuration thereof.
Figure 19 is a flowchart illustrating processing performed in the fourth embodiment.

### BEST MODE FOR CARRYING OUT THE INVENTION

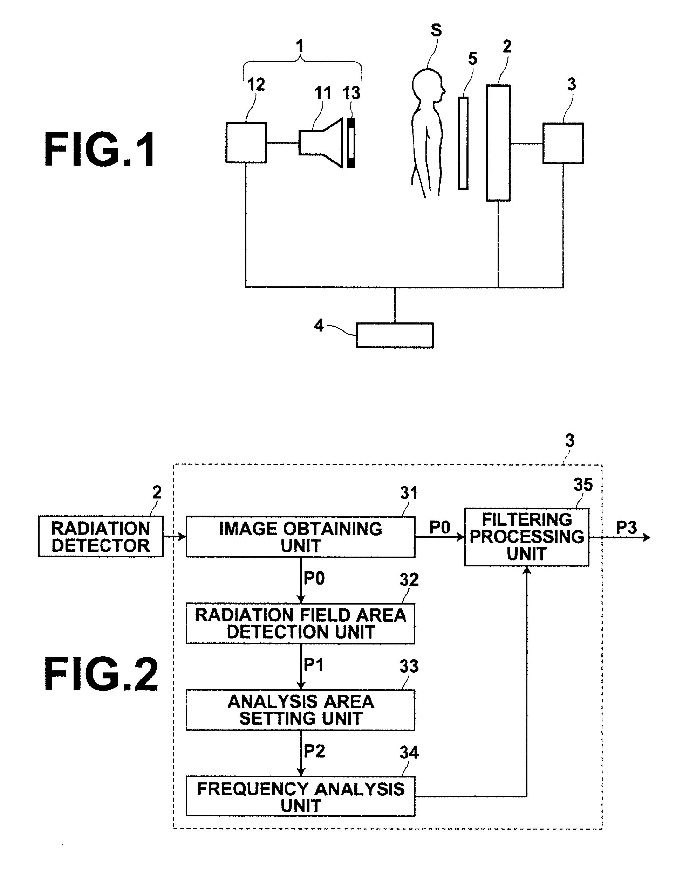

Hereinafter, embodiments of the present invention will be described with reference to the accompanying drawings. Figure 1 is a schematic block diagram of a radiation image diagnostic system to which a periodic pattern detection apparatus according to a first embodiment of the present invention is applied, illustrating a configuration thereof. As illustrated in Figure 1, the radiation image diagnostic system includes a radiation generation apparatus 1, a radiation detector 2, an image processing apparatus 3, an imaging control apparatus 4, and a grid 5, in which the radiation generation apparatus 1 and the radiation detector 2 are disposed opposite to each other across a subject S. The imaging control apparatus 4 is connected to each of the radiation generation apparatus 1, the radiation detector 2, and the image processing apparatus 3, while the radiation detector 2 is further connected to the image processing apparatus 3. In the present embodiment, it is assumed that the subject M wears a protector made of lead or the like in order to prevent radiation from being projected on an unnecessary region of the subject.

The radiation generation apparatus 1 includes a radiation tube 11 that emits radiation, a high voltage generator 12 that applies a tube voltage to the radiation tube 11, and a radiation field stop 13 for restricting the projection range of radiation emitted from the radiation tube 11, and is structured such that the radiation is projected to the subject S by narrowing the radiation field through control from the imaging control apparatus 4. The radiation emitted from the radiation tube 11 is projected to the subject S after the radiation field is narrowed down by the radiation field stop 13. Note that the setting of imaging conditions, such as the tube voltage, the tube current, the exposure time, and the like, and the operation control based on the imaging conditions are implemented by the imaging control apparatus 4.

The radiation detector 2 is used to store radiation image information formed of radiation transmitted through the subject S as an electrostatic latent image and to detect a radiation transmission distribution as a radiation image by reading out the stored electrostatic latent image. The radiation detector 2 may be of any configuration as long as it detects radiation and outputs the radiation as image information and it may be, for example, a TFT type solid-state detector or an optical readout type solid-state detector.

Further, the radiation image diagnostic system is of a structure in which the grid 5 is removably attachable between the subject S and the radiation detector 2, thereby allowing imaging both with and without the grid. Further, in the case of imaging with grid, various types of grids (grid ratios, grid patterns and the like) may be used. The grid 5 is of a structure in which lead that absorbs radiation and aluminum that transmits radiation are disposed alternately, for example, at a pitch of about 4/mm. The lead is disposed by slightly changing the inclination according to the position thereof, as required, so that the radiation passes through the aluminum and incident on the radiation detector 2.

The image processing apparatus 3 is a computer that includes a high-definition liquid crystal display that displays images and the like, a keyboard, a mouse, or the like that receives input from the user, and a main body provided with a CPU and a memory, a hard disk, a communication interface, and the like. The image processing apparatus 3 has a function to detect a periodic pattern arising from the grid from a radiation image and further to suppress the periodic pattern.

Figure 2 is a schematic block diagram of the image processing apparatus 3, illustrating a configuration thereof. As illustrated in Figure 2, the image processing apparatus 3 includes an image obtaining unit 31, a radiation field area detection unit 32, an analysis area setting unit 33, a frequency analysis unit 34, and a filtering processing unit 35.

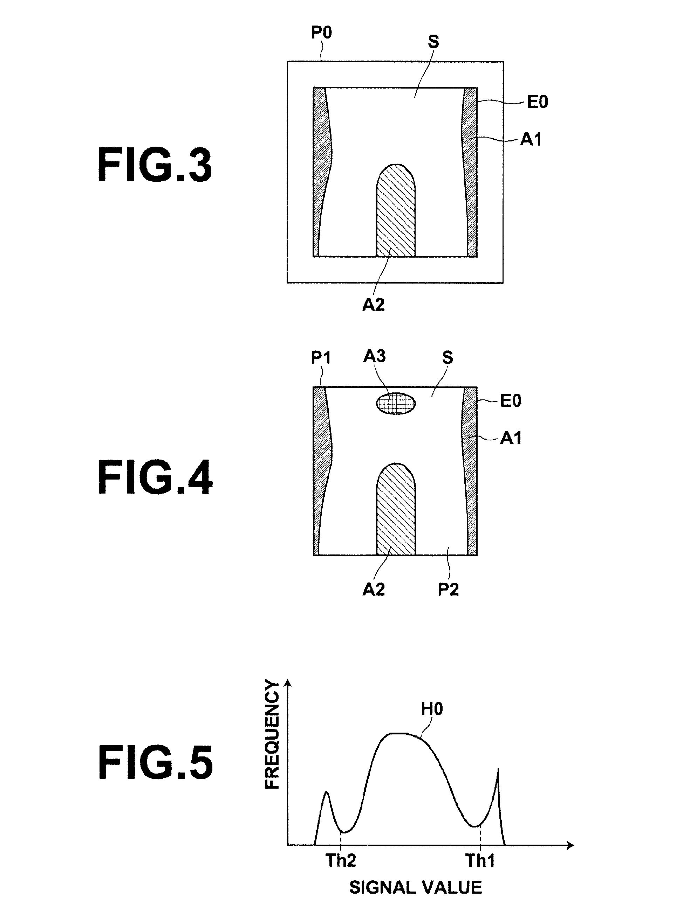

The image obtaining unit 31 obtains a radiation image P0 obtained by the radiation detector 2 as digital data. Figure 3 illustrates an example radiation image. As illustrated in Figure 3, the radiation image P0 includes a direct radiation section A1 which is an area where radiation was projected directly on the radiation detector 2 and a superabsorbent body area A2 which is an area of a protector for preventing radiation from being projected on an unnecessary region within an area enclosed by an edge E0 that defines the projection range of the radiation determined by the radiation field stop 13 (i.e., radiation field area), together with a transmission image of the subject S.

The radiation field area detection unit 32 detects a radiation field area where the radiation was projected through the radiation field stop 13 from the radiation image P0. For the detection of the radiation field area, for example, the method described in Japanese Unexamined Patent Publication No. 63(1988)-244029 may be used. The method described in Japanese Unexamined Patent Publication No. 63(1988)-244029 obtains edge candidate points presumed to be an edge portion of the radiation field, then, when a coordinate of an edge candidate point is taken as (x0, y0), obtains a curve, with (x0, y0) as a constant, represented by p = x0·cosθ+y0·sinθ for each edge candidate point, obtains, from the intersection points (p0, θ0) of these curves, straight lines defined as p0=x·cosθ0+y·sinθ0 in a rectangular coordinate system, and the area enclosed by these straight lines is detected as a radiation field area P1 where the radiation is projected. Note that the method of detecting the radiation field area is not limited to this, and any method may be used. This results in that the area enclosed by the edge E0 of the radiation field is detected as the radiation field area P1, as illustrated in Figure 4. Although, the radiation field is rectangular in the present embodiment, the radiation field may have any shape, including a circle, a polygon, and the like.

The analysis area setting unit 33 sets an area on which frequency analysis is to be performed by the frequency analysis unit 34 in the radiation field area P1 of the radiation image P0. More specifically, a direct radiation area, a superabsorbent body area, and a high noise area in the radiation field area P1 are detected as exclusion areas in which a periodic pattern arising from the grid is unlikely to present and sets an analysis area P2 by excluding the exclusion areas from the radiation field area P1. Hereinafter, the detection of direct radiation area, the superabsorbent body area, and the high noise area will be described.

As the direct radiation area A1 is an area in the radiation image P0 where the radiation is projected directly on the radiation detector 2 without transmitting through the subject S, it is the area with saturated density and blocked up in shadows. Therefore, the analysis area setting unit 33 first calculates a histogram of signal values of the radiation field area P1. Figure 5 illustrates a histogram of signal values of the radiation field area P1. In the histogram H0 illustrated in Figure 5, the horizontal axis represents the signal value and the vertical axis represents the frequency. Here, the direct radiation area A1 has an extremely high density. Therefore, the analysis area setting unit 33 sets, for example, a density corresponding to 95% of the maximum density in the distribution of the histogram to a threshold value Th1 and an area formed of pixel values whose signal values are greater than or equal to the threshold value Th1 is detected as the direct radiation area A1.

As the superabsorbent body area A2 is an area in the radiation image P0 where radiation is blocked by the protector, it is an area having very low density and blown out in highlights. Therefore, the analysis area setting unit 33 sets, for example, a density corresponding to 5% of the minimum density in the distribution of the histogram H0 to a threshold value Th2 and an area formed of pixel values whose signal values are greater than or equal to the threshold value Th2 is detected as the superabsorbent body area A2.

As for the high noise area, index values representing noise are calculated based on the information representing radiation dose when the radiation image P0 was obtained and a pixel whose index value is greater than or equal to a threshold value Th3 is detected as a high noise area, as described, for example, in Japanese Unexamined Patent Publication No. 2002-125153. In the present embodiment, for example, an area A3 shown in Figure 4 is detected as the high noise area.

Then, the analysis area setting unit 33 sets an area of the radiation field area P1 excluding the direct radiation area A1, the superabsorbent body area A2, and the high noise area A3 as the analysis area P2.

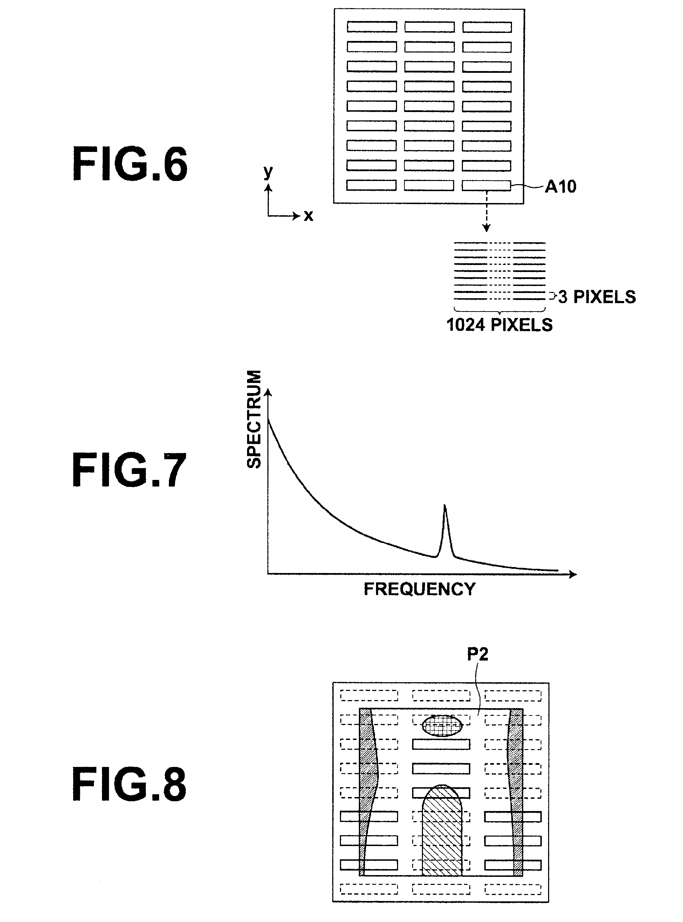

The frequency analysis unit 34 sets 3×9 subareas A10 in the radiation image P0, as shown in Figure 6. The subarea A10 includes 9 linear areas with a length of 1024 pixels in the x direction in Figure 6 at an interval of three pixels. Then, the frequency analysis unit 34 performs a Fourier transformation on an image signal of each linear area in the subarea A10 and calculates a frequency spectrum. Then, the frequency analysis unit 34 averages the 9 frequency spectra calculated in the subarea A10 and further averages the averaged frequency spectra calculated with respect to 3x9 subareas A10, thereby calculating a frequency spectrum of the radiation image P0 in the x direction. Likewise, a plurality of subareas is set in the y direction and a frequency spectrum in the y direction is calculated.

Figure 7 illustrates an example frequency spectrum. As illustrated in Figure 7, the obtained frequency spectrum is gradually decreased from low frequency toward high frequency with a peak at a certain frequency component. The frequency having the peak in the frequency spectrum calculated in the manner described above is the frequency of the periodic pattern arising from the grid. When performing imaging, if the pitch of the grid 5 is in the x direction, the peak frequency of periodic pattern arising from the grid appears in the frequency spectrum calculated in the x direction but does not appear in the y direction.

Here, in the present embodiment, the frequency analysis unit 34 performs the frequency analysis using only the analysis area P2. Consequently, in the present embodiment, not all of the 3x9 subareas A10 are used, and the frequency analysis is performed using only subareas whose areas are included in the analysis area P2 by more than or equal to 50% among 27 subareas A10, as illustrated in Figure 8. Note that subareas used in frequency analysis are indicated by the solid lines while those not used are indicated by the broken lines in Figure 8. As for the area ratio included in the analysis area P2 for the subarea used in frequency analysis is not limited to 50% and the ratio may be set to any value.

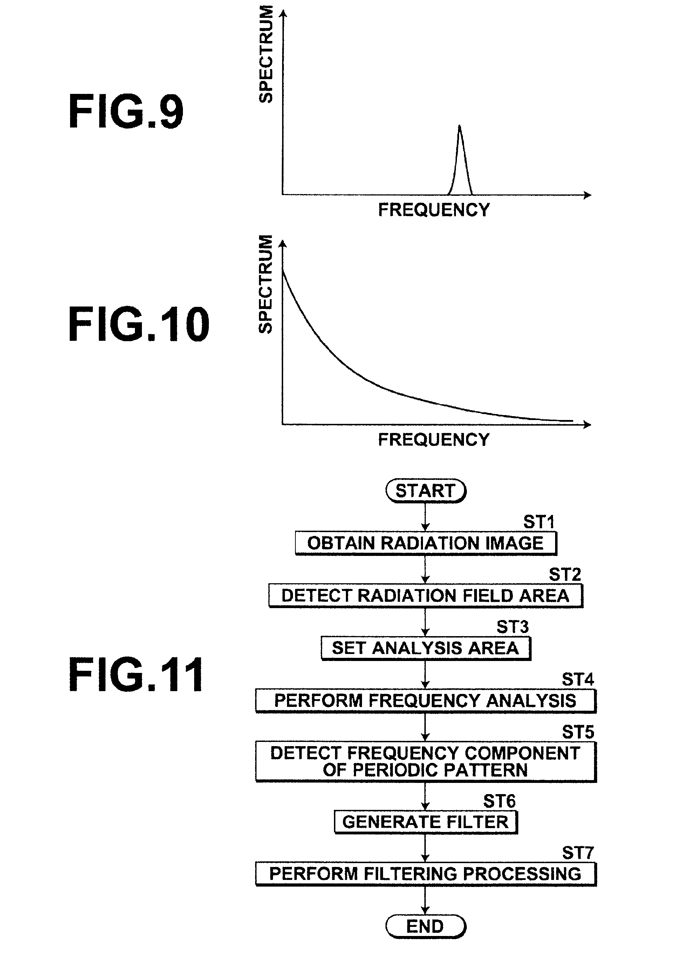

The filtering processing unit 35 generates a filter that extracts only the frequency component of the periodic pattern detected by the frequency analysis unit 35 and performs filtering processing on the radiation image P0 by the generated filter. The filtering-processed radiation image P0 changes to an image having only the frequency component of the periodic pattern, as illustrated in Figure 9. Then, the filtering processing unit 35 obtains a processed radiation image P3 by subtracting the filtering-processed radiation image from the radiation image P0. The frequency spectrum of the processed radiation image P3 appears that the frequency component of the periodic pattern arising from the grid is removed, as illustrated in Figure 10. The filtering processing may be performed not only on the radiation image P0 but also on the radiation field area P1.

Processing performed in the first embodiment will now be described. Figure 11 is a flowchart illustrating processing performed in the first embodiment. It is assumed here that the imaging of the subject S is completed. First, the image obtaining unit 31 obtains a radiation image P0 from the radiation detector 2 (step ST1), and the radiation field area detection unit 32 detects a radiation field area P1 from the radiation image P0 (step ST2) . Then, the analysis area setting unit 33 detects a direct radiation area, a superabsorbent body area, and a high noise area as exclusion areas in which a periodic pattern arising from the grid is unlikely to present, and sets an analysis area P2 by excluding the exclusion areas from the radiation field area P1 (step ST3).

Then, the frequency analysis unit 34 performs a frequency analysis only on the analysis area P2 (step ST4), and detects a frequency component of periodic pattern arising from the grid (step ST5). Then, the filtering processing unit 35 generates a filter that removes the frequency component of periodic pattern arising from the grid (step ST6), then obtains a processed radiation image P3 by performing filtering processing on the radiation image P0 by the generated filter (step ST7), and the processing is completed.

As described above, in the first embodiment, an analysis area P2 for frequency analysis is set by excluding areas in which a periodic pattern arising from the grid is unlikely to present in a radiation field area P1 and a frequency component of periodic pattern is detected by performing a frequency analysis on the analysis area P2. Consequently, even when a portion in which the amplitude of a period pattern is weak and a frequency response is small in a frequency spectrum obtained by the frequency analysis is included in the radiation field area P1, that portion is not used in the frequency analysis. Therefore, the frequency component of the periodic pattern may be detected accurately without being influenced by the areas in which a periodic pattern arising from the grid is unlikely to present.

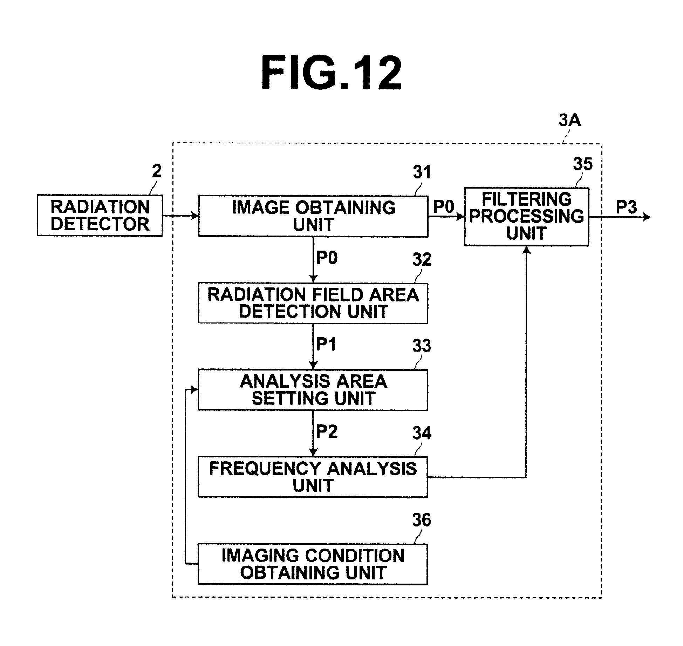

Next, a second embodiment of the present invention will be described. Figure 12 is a schematic block diagram of an image processing apparatus in a radiation image diagnostic system to which a periodic pattern detection apparatus according to a second embodiment of the present invention is applied, illustrating a configuration thereof. In the second embodiment, the components identical to those of the first embodiments are given the same reference numerals and will not be elaborated upon further here. The second embodiment differs from the first embodiment in that the image processing apparatus 3A includes an imaging condition obtaining unit 36 and the analysis area setting unit 33 sets the analysis area P2 according to the imaging conditions.

Here, if the value of tube currentxexposure time (i.e., mAs value) is high, it is highly likely that the density of the direct radiation area in a radiation image is saturated. Further, the noise is small as the dose of the radiation is large. Consequently, the analysis area setting unit 33 sets an analysis area P2 by detecting a direct radiation area A1 but not detecting a high nose area, if the mAs value is greater than or equal to the predetermined threshold value Th3. Conversely, if the mAs value is less than or equal to a predetermined threshold value Th4, the analysis area P2 is set without detecting a direct radiation area A1 while detecting a high noise area.

In the meantime, if the tube voltage is low, the contrast of a radiation image P0 to be obtained is high and the contrast of a periodic pattern arising from the grid is also sufficient. Consequently, the analysis area setting unit 33 sets the entire radiation field area P1 to the analysis area P2 without detecting the direct radiation area A1, the superabsorbent body area A2, and the high noise area A3, if the tube voltage is less than or equal to a predetermined threshold value Th5.

Further, the analysis area P2 may be set according to SID which is the distance from the radiation tube 11 to the imaging surface of the radiation detector 2. Here, the dose reaching the radiation detector 2 is decreased as the SID is increased, so that the analysis area P2 may be set by detecting a high nose area if the SID is greater than or equal to a predetermined threshold value Th6.

If comparison is made between imaging of a chest region and imaging of a hand, the latter requires less dose of radiation. Therefore, the analysis area P2 maybe set according to the imaging region of the subject S. More specifically, if the imaging region is a hand, the radiation dose is small, so that the analysis area P2 may be set by detecting a high noise area.

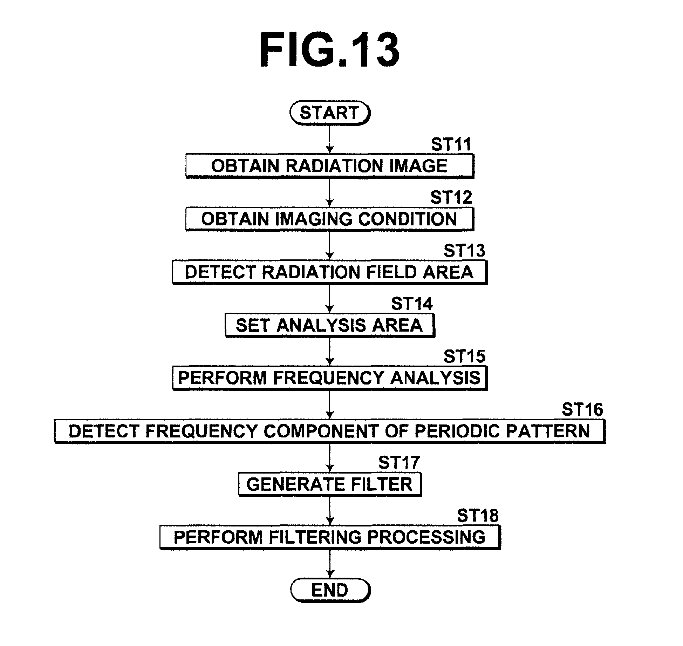

Next, processing performed in the second embodiment will be described. Figure 13 is a flowchart illustrating processing performed in the second embodiment. It is assumed here that the imaging of the subject S is completed. First, the image obtaining unit 31 obtains a radiation image P0 from the radiation detector 2 (step ST11), and the imaging condition obtaining unit 36 obtains imaging conditions (step ST12). Then, the radiation field area detection unit 32 detects a radiation field area P1 from the radiation image P0 (step ST13). Then, the analysis area setting unit 33 sets an analysis area P2 according to the imaging conditions (step ST14) .

Then, the frequency analysis unit 34 performs frequency analysis only on the analysis area P2 (step ST15), and detects a frequency component of a periodic pattern arising from the grid (step ST16). Then, the filtering processing unit 35 generates a filter that removes the frequency component of the periodic pattern arising from the grid (step ST17), obtains a processed radiation image P3 by performing filtering processing on the radiation image P0 by the generated filter (step ST18), and the processing is completed.

As described above, in the second embodiment, the analysis area is set according to the imaging conditions, so that the analysis area P2 may be set appropriately according to the circumstances in which the radiation image was obtained. Therefore, the frequency component of the periodic pattern may be detected more accurately.

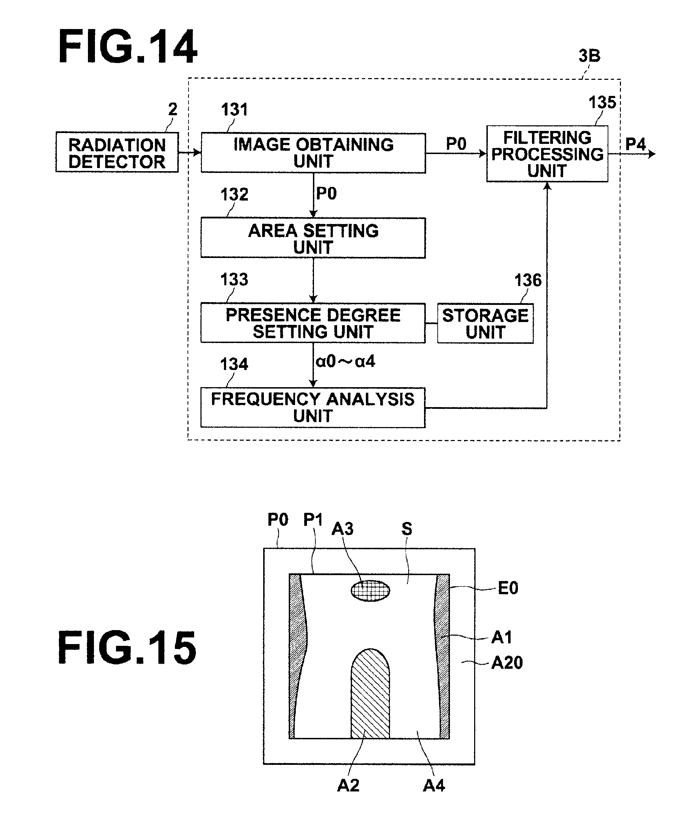

Next, a third embodiment of the present invention will be described. Figure 14 is a schematic block diagram of an image processing apparatus in a radiation image diagnostic system to which a periodic pattern detection apparatus according to a third embodiment of the present invention is applied, illustrating a configuration thereof. In the third embodiment, the components identical to those of the first embodiments are given the same reference numerals and will not be elaborated upon further here.

As illustrated in Figure 14, the image processing apparatus 3B includes an image obtaining unit 131, an area setting unit 132, a presence degree setting unit 133, a frequency analysis unit 134, a filtering processing unit 135, and a storage unit 136. Note that the area setting unit 132, the presence degree setting unit 133, the frequency analysis unit 134, and the filtering processing unit 135 constitute a second periodic pattern detection apparatus of the present invention.

The image obtaining unit 131 obtains a radiation image P0 obtained by the radiation detector 2 as digital data. Figure 15 illustrates an example radiation image obtained in the third embodiment. As illustrated in Figure 15, the radiation image P0 includes a direct radiation section A1 which is an area where radiation was projected directly on the radiation detector 2 and a superabsorbent body area A2 which is an area of a protector for preventing the radiation from being projected on an unnecessary region, and a high noise area A3, within an area enclosed by an edge E0 that defines the projection range of the radiation determined by the radiation field stop 13 (i.e., radiation field area), together with a transmission image of the subject S, an outside radiation field area A20, and an area A4 other than these areas.

The area setting unit 132 sets 3×9 subareas A10 in the radiation image P0, as shown in Figure 6, as in the first embodiment. Here, the subarea A10 includes 9 linear areas with a length of 1024 pixels in the x direction in Figure 6 at an interval of three pixels and is a rectangular region having a long side in the x direction. In the third embodiment, the area setting unit 132 also sets subareas having a long side in the y direction.

The presence degree setting unit 133 sets a presence degree of a periodic pattern arising from the grid 5 to each of a plurality of subareas A10 (including the y direction, the same applies hereinafter) . Hereinafter, the setting of the presence degree will be described. First, the presence degree setting unit 133 detects the radiation field area P1, the direct radiation area A1, the superabsorbent body area A2, and the high noise area A3 from the radiation image P0. The detection of the radiation field area P1, the direct radiation area A1, the superabsorbent body area A2, and the high noise area A3 is performed as in the first embodiment. The outside radiation field area A20 is an area of the radiation image P0 other than the radiation field area P1.

Then, the presence degree setting unit 133 sets periodic pattern presence degrees to the outside radiation field area A20, the direct radiation area A1, the superabsorbent body area A2, and the high noise area A3, as well as an area other than those areas (hereinafter, referred to as the area A4) in the radiation image P0. More specifically, predetermined constants α0, α1, α2, α3, α4 are set to the outside radiation field area A20, the direct radiation area A1, the superabsorbent body area A2, the high noise area A3, and the area A4 respectively as the presence degrees. Here, a value of 1 is set to the α4 while values not less than 0 and not greater than 1 are set to the α0 to α3. For example, α0=0, α1=0.5, α2=0.5, α3=0.5 are set as the presence degrees. It is assumed here that the presence degrees according to the region are predetermined and stored in the storage unit 136.

The frequency analysis unit 134 performs a Fourier transformation on an image signal of each linear area in the subarea A10 set by the area setting unit 132 and calculates a frequency spectrum. Then, the frequency analysis unit 134 averages the 9 frequency spectra calculated in the subarea A10 and further performs weighted average on the 27 averaged frequency spectra calculated with respect to 3×9 subareas A10 according to the α0 to α4, thereby calculating a frequency spectrum of the radiation image P0 in the x direction. Also, in the y direction, frequency spectra of a plurality of subareas are calculated and the plurality of frequency spectra are weighted averaged according to the α0 to α4, as in the x direction, thereby calculating a frequency spectrum of the radiation image P0 in the y direction. The frequency spectrum is identical to that shown in Figure 7.

Note that there may be a case where the subarea extends to a plurality of regions of the outside radiation field area A20, the direct radiation area A1, the superabsorbent body area A2, and the high noise area A3. In this case, the weight for the subarea A10 is a multiplied value of the presence degrees of the areas where the subarea A10 belongs. For example, if a subarea A10 belongs to both the superabsorbent body area A2 and the high noise area A3, the weight of the subarea A10 is a value obtained by multiplying the presence degree α2 of the superabsorbent body area A2 and the presence degree α3 of the high noise area A3 (i.e., α2×α3).

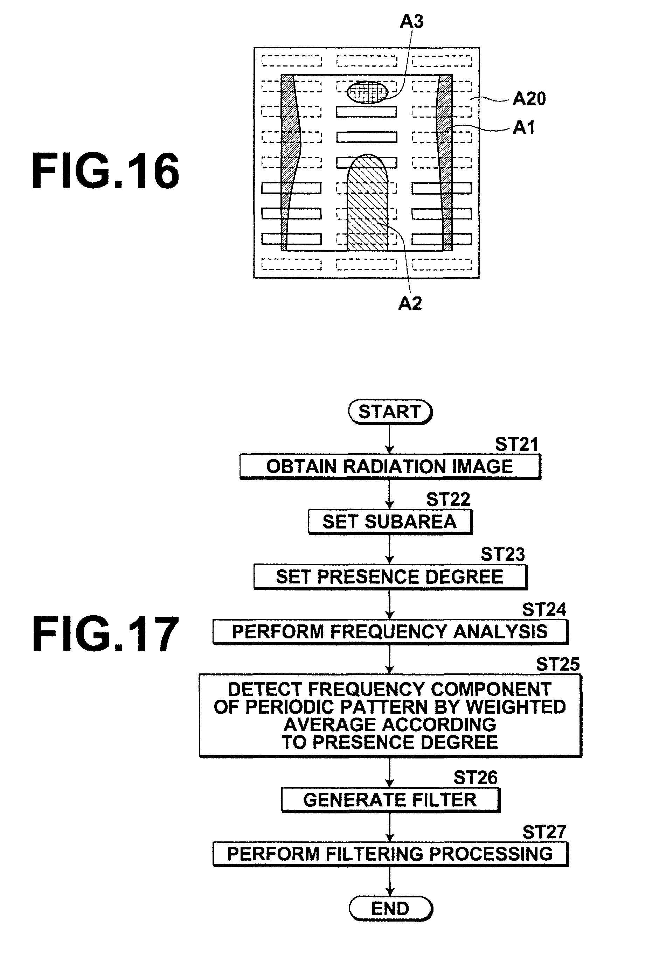

Here, in the third embodiment, weighting is performed on each of the averaged frequency spectra calculated for 27 subareas A10 according to the presence degrees α0 to α4. Consequently, as illustrated in Figure 16, among the 27 subareas A10, subareas A10 which belong to the area A4 as indicated by the solid lines are weighted by α4 (e.g., value of 1) while the subareas A10 whose areas belong to the outside radiation field area A20, the direct radiation area A1, the superabsorbent body area A2, or the high noise area A3 by 50% or more as indicated by the broken lines are weighted by α0 to α4, which are values not greater than 1, respectively according to the areas they belong. As for the area ratio included in the outside radiation field area A20, the direct radiation area A1, the superabsorbent body area A2, or the high noise area A3 is not limited to 50% and the ratio may be set to any value.

The filtering processing unit 135 generates a filter that extracts only the frequency component of the periodic pattern detected by the frequency analysis unit 134 and performs filtering processing on the radiation image P0 by the generated filter. The filtering-processed radiation image P0 changes to an image having only the frequency component of the periodic pattern, as illustrated in Figure 9. Then, the filtering processing unit 135 obtains a processed radiation image P4 by subtracting the filtering-processed radiation image from the radiation image P0. The frequency spectrum of the processed radiation image P4 appears that the frequency component of the periodic pattern arising from the grid is removed, as illustrated in Figure 10.

Next, processing performed in the third embodiment will now be described. Figure 17 is a flowchart illustrating processing performed in the third embodiment. It is assumed here that the imaging of the subject S is completed. First, the image obtaining unit 131 obtains a radiation image P0 from the radiation detector 2 (step ST21), and the area setting unit 132 sets subareas in the radiation image P0 for detecting a frequency pattern (step ST22). Then, the presence degree setting unit 133 sets presence degrees α0 to α4 to the outside radiation field area A20, the direct radiation area A1, the superabsorbent body area A2, the high noise area A3, and the area A4 other than these areas (step S23).

Then, the frequency analysis unit 134 performs a frequency analysis on each subarea A10 (step ST24), and detects a frequency component of a periodic pattern arising from the grid by performing a weighted average on frequency spectra calculated for the respective subareas A10 according the presence degrees α0 to α4 (step ST25). Then, the filtering processing unit 135 generates a filter that removes the frequency component of the periodic pattern arising from the grid (step ST26), obtains a processed radiation image P4 by performing filtering processing on the radiation image P0 by the generated filter (step ST27), and the processing is completed.

As described above, in the second embodiment, a plurality of subareas A10 is set throughout the radiation image obtained by performing imaging using a grid for removing a scattered component of the radiation, presence degrees α0 to α4 of period pattern arising from the grid are set to the respective subareas A10, and a frequency component of a periodic pattern in the radiation image P0 is detected by performing a weighted average on the frequency spectra of a plurality of subareas A10 according to the presence degrees α0 to α4. Consequently, even when a portion in which the amplitude of a period pattern is weak and a frequency response is small in a frequency spectrum obtained by the frequency analysis is included in the radiation image, such as the outside radiation field area A20, the direct radiation area A1, the superabsorbent body area A2, or the high noise area A3, a small weight is set to that portion when the frequency component is detected. Therefore, the frequency component of the periodic pattern may be detected accurately without being influenced by the areas in which a periodic pattern is unlikely to present.

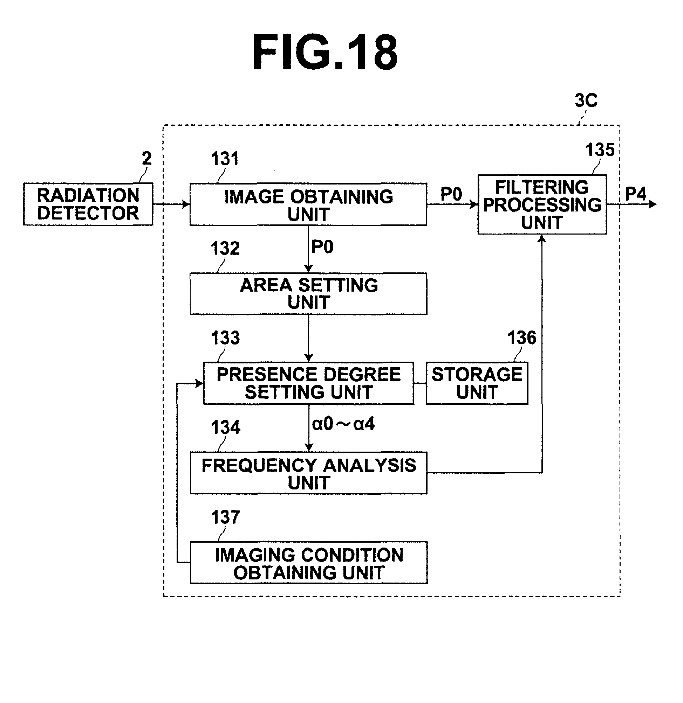

Next, a fourth embodiment of the present invention will be described. Figure 18 is a schematic block diagram of an image processing apparatus in a radiation image diagnostic system to which a periodic pattern detection apparatus according to a fourth embodiment of the present invention is applied, illustrating a configuration thereof. In the fourth embodiment, the components identical to those of the third embodiments are given the same reference numerals and will not be elaborated upon further here. The fourth embodiment differs from the third embodiment in that the image processing apparatus 3C includes an imaging condition obtaining unit 137 that obtains imaging conditions from the imaging control unit 4 and the presence degree setting unit 133 sets the presence degrees α0 to α4 according to the imaging conditions.

Here, if the value of tube currentxexposure time (i.e., mAs value) is high, it is highly likely that the density of the direct radiation area in a radiation image is saturated. Further, the noise is small as the dose of the radiation is large. Consequently, the presence degree setting unit 133 sets the presence degree of the direct radiation area A1 to a small value if the mAs value is greater than or equal to the predetermined threshold value Th4. More specifically, the presence degree α1 set regardless of the imaging conditions is multiplied by a constant β which is less than 1, thereby setting the presence degree to a smaller value. Conversely, if the mAs value is less than or equal to the predetermined threshold value Th5, the noise included in the radiation image P0 becomes noticeable, so that the presence degree of the high noise area A3 is set to low.

Further, the presence degree may be set according to SID which is the distance from the radiation tube 11 to the imaging surface of the radiation detector 2. Here, the dose reaching the radiation detector 2 is decreased as the SID is increased, so that the presence degree of the high noise area A3 is set to low, if the SID is greater than or equal to the predetermined threshold value Th5.

If comparison is made between imaging of a chest region and imaging of a hand, the latter requires less dose of radiation. Therefore, the presence degree may be set according to the imaging region of the subject S. More specifically, if the imaging region is a hand, the radiation dose is small, so that the presence degree of the high noise area A3 is set to low.

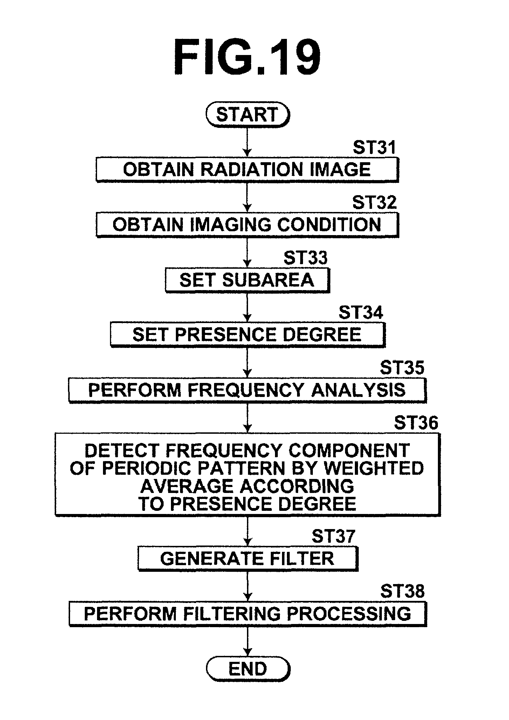

Next, processing performed in the fourth embodiment will be described. Figure 19 is a flowchart illustrating processing performed in the fourth embodiment. It is assumed here that the imaging of the subject S is completed. First, the image obtaining unit 131 obtains a radiation image P0 from the radiation detector 2 (step ST31), and the imaging condition obtaining unit 137 obtains imaging conditions (step ST32) . Then the area setting unit 132 sets subareas in the radiation image P0 for detecting a frequency pattern (step ST33) . Then, the presence degree setting unit 133 sets presence degrees α0 to α4 to the outside radiation field area A20, the direct radiation area A1, the superabsorbent body area A2, the high noise area A3, and the area A4 other than these areas (step S34).

Then, the frequency analysis unit 134 performs a frequency analysis on each subarea A10 (step ST35), and detects a frequency component of a periodic pattern arising from the grid by performing a weighted average on frequency spectra calculated for the respective subareas A10 according the presence degrees α0 to α4 (step ST36). Then, the filtering processing unit 135 generates a filter that removes the frequency component of the periodic pattern arising from the grid (step ST37), obtains a processed radiation image P4 by performing filtering processing on the radiation image P0 by the generated filter (step ST38), and the processing is completed.

As described above, in the fourth embodiment, the presence degrees are set according to the imaging conditions, so that the presence degrees may be set appropriately according to the circumstances in which the radiation image was obtained. Therefore, the frequency component of the periodic pattern may be detected more accurately.

In the first and the second embodiments described above, the analysis area is set by excluding the direct radiation area, the superabsorbent body area, and the high noise area, but it is not necessary to exclude all of the direct radiation area, the superabsorbent body area, and the high noise area, and the analysis area may be set by excluding at least one of them. In particular, the analysis area may be set by excluding only the high noise area.

Further, in the first and the second embodiments described above, the frequency analysis is performed using subareas included in the analysis area P2 by more than or equal to a predetermined ratio among the 3×9 subareas, but the frequency analysis may be performed using subareas entirely included in the analysis area P2. Further, frequency analysis may be performed by newly setting a subarea in the analysis area P2.

Further, in the third and the fourth embodiments described above, the presence degrees are set by detecting the outside radiation field area A20, the direct radiation area A1, the superabsorbent body area A2, and the high noise area A3, but it is not necessary to detect all of the outside radiation field area A20, the direct radiation area A1, the superabsorbent body area A2, and the high noise area A3, and the presence degrees may be set by detecting at least one of them. In particular, the presence degree may be set by excluding only the high noise area A3.

Still further, in the third and the fourth embodiments described above, the presence degrees α0 to α3 are preset, but probabilities of being the outside radiation field area A20, the direct radiation area A1, the superabsorbent body area A2, and the high noise area A3 may be set as the presence degrees α0 to α3. More specifically, ratios of the areas of the outside radiation field area A20, the direct radiation area A1, the superabsorbent body area A2, and the high noise area A3 to the area of the subarea A10 may be set as the presence degrees α0 to α3. For example, if the area ratio of the outside radiation field area A20 is 40% in a certain subarea A10, the presence degree may be set as α0=0.4.

Further, with respect to the superabsorbent body area A2, an arrangement may be adopted in which signal values from 5% to 1% of the minimum density are allocated to the probabilities 0% to 100%, an average value of the probabilities of being the superabsorbent body area for the pixel value of each pixel included in the subarea is calculated, and the average value is used as the presence degree α2. Still further, with respect to the high noise area A3, an arrangement may be adopted in which a pixel whose index value representing noise corresponds to the threshold value Th6 is defined as noise probability 0% and a pixel corresponding to a threshold value Th7 which is greater than the threshold Th6 is defined as noise probability 100%, an average value of index values representing noise with respect to each pixel included in the subarea A10 is obtained, and the average value is used as the presence degree α3. If the index value falls in the range from the threshold value Th6 to the threshold value Th7, the noise probability may be calculated by linear interpolation.

Still further, in the first to the fourth embodiments, the radiation image of the subject S is obtained using the radiation detector 2, but the radiation image may be obtained using a storage phosphor sheet that makes use of a storage phosphor body that stores, by receiving radiation, a part of the energy of the radiation and, thereafter, emits stimulated emission light by receiving excitation light, such as visible light, laser light, or the like. In the case where the storage phosphor sheet is used, a radiation image is obtained by tentatively storing and recording radiation image information by projecting radiation transmitted through the subject on the storage phosphor sheet, generating stimulated emission light by projecting excitation light on the storage phosphor sheet, and photoelectrically converting the stimulated emission light.

## Claims

1. A periodic pattern detection apparatus, comprising:
a radiation field area detection means that detects, from a radiation image obtained by performing imaging using a radiation field stop and a grid that removes a scattered component of radiation, a radiation field area corresponding to the radiation field stop;
an analysis area setting means that sets an analysis area for performing a frequency analysis by excluding an area in the radiation field area where a periodic pattern is unlikely to present; and
a frequency analysis means that detects a frequency component of the periodic pattern by performing a frequency analysis on the analysis area.

2. The periodic pattern detection apparatus as claimed in claim 1, wherein the area where the periodic pattern is unlikely to present is at least one of a direct radiation area, a superabsorbent body area, and a high noise area.

3. The periodic pattern detection apparatus as claimed in claim 1 or 2, wherein the analysis area setting means sets the analysis area according to an imaging condition at the time of obtaining the radiation image.

4. The periodic pattern detection apparatus as claimed in claim 1, wherein the area where the periodic pattern is unlikely to present is a high noise area.

5. The periodic pattern detection apparatus as claimed in any of claims 1 to 4, wherein the apparatus further comprises a processing means that obtains a processed radiation image by removing the frequency component of the periodic pattern.

6. A periodic pattern detection method, comprising the steps of:
detecting, from a radiation image obtained by performing imaging using a radiation field stop and a grid that removes a scattered component of radiation, a radiation field area corresponding to the radiation field stop;
setting an analysis area for performing a frequency analysis by excluding an area in the radiation field area where a periodic pattern is unlikely to present; and
detecting a frequency component of the periodic pattern by performing a frequency analysis on the analysis area.

7. A periodic pattern detection apparatus, comprising:
an area setting means that sets a plurality of subareas throughout a radiation image obtained by performing imaging using a grid that removes a scattered component of radiation;
a presence degree setting means that sets a presence degree of a periodic pattern arising from the grid to each of the plurality of subareas; and
a frequency analysis means that calculates a plurality of frequency spectra for each of the plurality of subareas by performing a frequency analysis on each of the plurality of subareas and detects a frequency component of the periodic pattern by weighted averaging the plurality of frequency spectra according to the presence degree.

8. The periodic pattern detection apparatus as claimed in claim 7, wherein, if a subarea belongs to at least one of an outside radiation field area, a direct radiation area, a superabsorbent body area, and a high noise area in the radiation image, the presence degree setting means is a means that sets the presence degree of the subarea smaller than that of a subarea which belongs to an area other than the outside radiation field area, the direct radiation area, the superabsorbent body area, and the high noise area.

9. The periodic pattern detection apparatus as claimed in claim 7, wherein, if a subarea belongs to a high noise area, the presence degree setting means is a means that sets the presence degree of the subarea smaller than that of a subarea which belongs to an area other than the high noise area.

10. The periodic pattern detection apparatus as claimed in any of claims 7 to 9, wherein the presence degree setting means is a means that sets the presence degree according to an imaging condition at the time of obtaining the radiation image.

11. The periodic pattern detection apparatus as claimed in any of claims 7 to 10, wherein the apparatus further comprises a processing means that obtains a processed radiation image by removing the frequency component of the periodic pattern.

12. A periodic pattern detection method, comprising the steps of:
setting a plurality of subareas throughout a radiation image obtained by performing imaging using a grid that removes a scattered component of radiation;
setting a presence degree of a periodic pattern arising from the grid to each of the plurality of subareas;
calculating a plurality of frequency spectra for each of the plurality of subareas by performing a frequency analysis on each of the plurality of subareas; and
detecting a frequency component of the periodic pattern by weighted averaging the plurality of frequency spectra according to the presence degree.
